# EUROPEAN PATENT SPECIFICATION

(11) **EP 4 028 543 B1**
(45) Date of publication and mention of the grant of the patent: **12.06.2024**
(21) Application number: 20768411.9
(22) Date of filing: 04.09.2020
(51) Int. Cl.: C12Q 1/68

(54) **METHOD FOR PREPARING AN RNA SAMPLE FOR SEQUENCING AND KIT THEREOF**
VERFAHREN ZUR HERSTELLUNG EINER RNA-PROBE ZUR SEQUENZIERUNG UND KIT DAFÜR
PROCÉDÉ DE PRÉPARATION D'UN ÉCHANTILLON D'ARN POUR SÉQUENÇAGE ET KIT ASSOCIÉ

(30) Priority: 09.09.2019 IT 201900015914
(43) Date of publication of application: 20.07.2022
(73) Proprietor: Immagina Biotechnology S.r.l., 38123 Trento (IT)
(72) Inventor: DEL PIANO, Alessia, 38121 Trento (IT); FIRRITO, Claudia, 38123 Trento (IT); CLAMER, Massimiliano, 38123 Trento (IT)
(74) Representative: Freyria Fava, Cristina
(86) International application number: PCT/IB2020/058259
(87) International publication number: WO 2021/048720

(56) References cited:
- WO-A1-2018/175258
- US-A1- 2012 156 730
- US-A1- 2014 057 322
- US-A1- 2018 362 968
- SHOZO HONDA ET AL: "Selective amplification and sequencing of cyclic phosphate-containing RNAs by the cP-RNA-seq method", NATURE PROTOCOLS, vol. 11, no. 3, 11 February 2016 (2016-02-11), pages 476-489, XP055684897, GB ISSN: 1754-2189, DOI: 10.1038/nprot.2016.025
- A. T. LAMM ET AL: "Multimodal RNA-seq using single-strand, double-strand, and CircLigase-based capture yields a refined and extended description of the C. elegans transcriptome", GENOME RESEARCH, vol. 21, no. 2, 22 December 2010 (2010-12-22), pages 265-275, XP055221889, US ISSN: 1088-9051, DOI: 10.1101/gr.108845.110
- K. SCHUTZ ET AL: "Capture and sequence analysis of RNAs with terminal 2',3'-cyclic phosphates", RNA, vol. 16, no. 3, 1 March 2010 (2010-03-01), - 1 March 2010 (2010-03-01), pages 621-631, XP055357492, US ISSN: 1355-8382, DOI: 10.1261/rna.1934910
- SONJA PETKOVIC ET AL: "RNA circularization strategies in vivo and in vitro", NUCLEIC ACIDS RESEARCH, vol. 43, no. 4, 6 February 2015 (2015-02-06), pages 2454-2465, XP055488942, ISSN: 0305-1048, DOI: 10.1093/nar/gkv045
- GUBLER U ET AL: "A simple and very efficient method for generating cDNA libraries", GENE, ELSEVIER AMSTERDAM, NL, vol. 25, no. 2-3, 1 November 1983 (1983-11-01), pages 263-269, XP023599413, ISSN: 0378-1119, DOI: 10.1016/0378-1119(83)90230-5 [retrieved on 1983-11-01]

## Description

### Field of the invention

The present description concerns a novel method for preparing an RNA sample for sequencing and a kit for carrying out such method.

### Background

RNA-protein interactions play a fundamental role in controlling crucial aspects of cell biology, from mRNA transcription, pre-mRNA splicing and RNA signaling function to translation and protein localization¹. Given the importance of understanding such biological processes, several efforts have been spent to develop methods to study and characterize these interactions, from chemical labelling of RNA and proteins² to whole genome high-throughput sequencing of RNA footprints.³⁻⁶ However, sequencing approaches generally suffer from several limitations during sample preparation, such as extensive manipulation steps, PCR amplification and the inability to selectively capture RNA sequences bearing a phosphate or a 2',3'-cyclic phosphate group at the 3' end (3'-P/cP), thus resulting in reduced output accuracy⁷. This leads to library cross-reactivity with undesired RNA targets, high background noise and poor library quality, hindering important biological information of 3'-P/cP-terminated RNA products. The 3'-P/cP is generated by enzymatic cleavage and 3'-P/cP RNAs have a key role in many disease states (such as cancer and amyotrophic lateral sclerosis^{8,9}), biological processes (such as the unfolding protein response¹⁰, stress granule production⁸, RNA metabolism¹¹, rRNA and tRNA biogenesis¹² and mRNA splicing¹³), and biological functions (such as neuronal survival¹⁴ and inflammatory response¹⁵). Although the phosphate signature at the 3' end is an important functional marker, most sequencing pipelines do not preserve this chemical feature during library preparation. Few methods are available for the detection of 3'-P or 3'-cP, but they either only allow indirect detection of 3'-P¹⁶, or are exclusively selective for 3'-cP^{16,17}. Additionally, these protocols are laborious and time consuming, involving PCR amplification steps, which can result in uneven sequence coverage or sequencing errors (e.g. inside repetitive regions).

From a technical perspective, many RNA footprinting techniques employ endoribonucleases to characterize RNA-protein interaction , large RNA-protein complexes¹⁸ or the interaction of small molecules¹⁹ with RNA. An experimental setting that is strongly affected by the lack of available library preparation protocols able to selectively capture 3'-P termini is ribosome profiling (Ribo-seq), an RNA footprinting method based on the deep sequencing of 25-35 nt-long ribosome-protected fragments (RPFs), namely the mRNA fragments generated after nuclease digestion of unprotected single-stranded RNA. Providing positional information of ribosomes along transcripts captured in a particular moment, this technique represents a powerful approach to study the biology of protein synthesis²⁰. Current protocols for ribosome profiling involve many sequential steps and are based on the Illumina sequencing platform. In particular, after the isolation of RPFs, two alternative library preparation workflows are available to date: (i) workflow based on the sequential steps of adaptor ligation at the 3' end of RPFs, cDNA synthesis, circularization and PCR amplification, with a total of four gel extraction steps²¹; (ii) ligation-independent workflow for low-input material, for which commercial products are available, involving the sequential steps of RNA 3' polyadenylation, cDNA synthesis with template switching and PCR amplification²², and requiring a gel extraction step. Major drawbacks of the available protocols are represented by (i) PCR amplification biases and (ii) the lack of preservation of 3'-P/cP terminus (which provide a signature of effective digestion) with subsequent underrepresentation of 3'-P/cP-bearing RNA species (representing the actual RPFs) in sequencing datasets. Indeed, both workflows require a dephosphorylation step before adaptor ligation³ or polyadenylation. This manipulation step lowers the level of specify in ligation reactions, resulting in the capture of any short RNA molecules endowed with -OH groups at their 3' terminus.

On top of that, recent studies have revealed important differences within biological fluids (cord-blood plasma, bronchoalveolar lavage, adult blood plasma, parotid saliva, ovarian follicle fluid, serum, amniotic fluid, seminal plasma, urine, bile, submandibular/sublingual saliva, cerebrospinal fluid)in the relative amount and type of small RNA populations such as tRNA derived RNAs, piwi-interacting RNAs, Y RNAs. Importantly, some of them are known to have a 3'P or 2',3'-cP and have been associated to cancer, neuro- and immunological disorders²³. In this clinical scenario, these RNA species can have a potential role as biomarkers, with a predictive and/or prognostic significance in patient stratification^{24,25}

US 2014/057322 discloses a method of amplifying RNA in a sample comprising forming an adapter nucleic acid-RNA fusion construct by ligating the RNA to an adapter nucleic acid; forming a circular adapter nucleic acid-RNA fusion construct by self-ligating the adapter nucleic acid-RNA fusion construct; and forming an amplified product by incubating the circular adapter nucleic acid-RNA fusion construct, and primers.

There is therefore the need for new methods of preparing an RNA sample for sequencing that are free from the drawbacks of the known methods.

### Summary of the invention

The object of this disclosure is to provide a novel method for preparing an RNA sample for sequencing and a kit for implementing such a method.

According to the invention, the above object is achieved thanks to the subject matter recalled specifically in the ensuing claims, which are understood as forming an integral part of this disclosure.

The present invention concerns an in vitro method for preparing at least one RNA molecule contained in a biological sample for sequencing comprising the following steps:
(i) obtaining a biological sample comprising at least one RNA molecule, wherein the at least one RNA molecule bears a phosphate or a 2',3'-cyclic phosphate group at 3' end;
(ii) phosphorylating the at least one RNA molecule at the 5' end, thus introducing a phosphate group at the 5' end of the at least one RNA molecule, and obtaining at least one RNA molecule phosphorylated at both ends;
(iii) ligating the 3' end of the at least one phosphorylated RNA molecule to the 5' end of a random RNA linker, wherein the random RNA linker bears a -OH group at both ends, obtaining at least one first ligation product;
(iv) self-ligating the at least one first ligation product to form at least one circular RNA molecule, wherein the at least one circular RNA molecule is mixed with linear RNA molecules;
(v) digesting the linear RNA molecules; and
(vi) subjecting the at least one circular RNA molecule to reverse transcription rolling circular amplification, obtaining at least one single-stranded cDNA molecule, wherein the at least one single-stranded cDNA molecule carries at least 1, preferably between 2 and 500, copies of the sequence of the at least one RNA molecule;
   wherein the at least one single-stranded cDNA molecule carrying at least 1 copy of the sequence of the at least one RNA molecule is suitable for sequencing, preferably single molecule sequencing.

The instant method is PCR-free and can be applied to any 3'-P/cP-terminated RNA footprint. The method object of the instant application, named CircAID-p-seq, is a fast cDNA sequencing protocol for low-input biological samples optimized for nanopore sequencing. This method overcomes some of the limitations that traditionally have been plaguing RNA footprinting, such as a time-consuming protocols and PCR biases, and provide powerful pipeline for deep sequencing of 3'-P/cP-bearing RNA fragments with the Oxford Nanopore platform, thus enabling real-time single-molecule detection of biologically relevant RNA species.

In a further embodiment the present invention concerns a kit for carrying out the method for preparing at least one RNA molecule contained in a biological sample for sequencing (as herein disclosed), wherein the kit comprises a random RNA linker, and a first ligase enzyme, an exoribonuclease, and optionally a second ligase enzyme, wherein:
(i) the random RNA linker bears -OH group at both ends;
(ii) the ligase enzyme is suitable to ligate the 3' end of an RNA molecule, bearing a phosphate or a 2',3'-cyclic phosphate group at the 3' end and a phosphate group at the 5' end, to the 5' end of the random RNA linker bearing a hydroxyl group at the 3' end;
(iii) the exoribonuclease is suitable to
(iv) the second ligase enzyme is suitable to circularize a ligation product obtained by ligation of the random RNA linker to the RNA molecule.

### Brief description of the drawings

The invention will now be described in detail, purely by way of an illustrative and non-limiting example and, with reference to the accompanying drawings, wherein:
- **Figure 1****.** A) TBE-urea PAGE analysis of fragments resulting from RNAse I digestion with or without polyadenylation treatment. B) Schematic representation of the CircAID-p-seq workflow. C) TBE-urea PAGE analysis of all CircAID-p-seq steps.
- **Figure 2****.** Direct cDNA sequencing of the circGFP-linkerR library. A) Length distribution of the sequencing reads; B) Representative consensus sequence. A single, base-called read is split into its individual repeats, then aligned to each other to generate a consensus sequence.
- **Figure 3****.** A) Representative pictures of GFP-transfected HEK293T cells. B) Length distribution of GFP fragments detected with BLASTn. C) BLASTn alignment of sequencing reads to the reference GFP sequence.
- **Figure 4****.** Representative consensus sequence generated by 2 (up) and 3 repeats (down) obtained from two different reads.

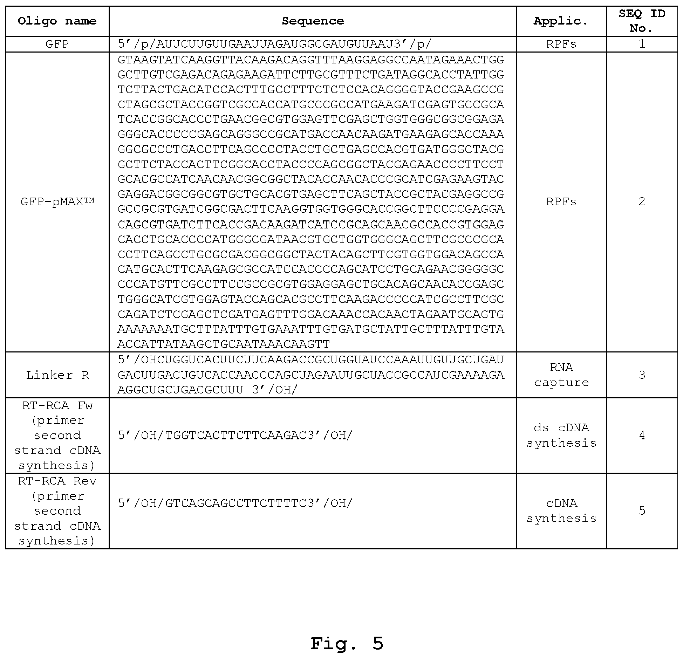

- **Figure 5****.** Nucleotide sequences.

### Detailed description of the invention

In the following description, numerous specific details are given to provide a thorough understanding of the embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

The instant invention concerns a novel method for preparing at least one RNA molecule contained in a biological sample for sequencing comprising the following steps:
(i) obtaining a biological sample comprising at least one RNA molecule, wherein the at least one RNA molecule bears a phosphate or a 2',3'-cyclic phosphate group at 3' end;
(ii) phosphorylating the at least one RNA molecule at the 5' end, thus introducing a phosphate group at the 5' end of the at least one RNA molecule and obtaining at least one RNA molecule phosphorylated at both ends;
(iii) ligating the 3' end of the at least one phosphorylated RNA molecule to the 5' end of a random RNA linker, wherein the random RNA linker bears a -OH group at both ends, obtaining at least one first ligation product;
   enzymatically digest linear RNA molecules; and
(iv) self-ligating the at least one first ligation product to form at least one circular RNA molecule, wherein the at least one circular RNA molecule is mixed with linear RNA molecules;
(v) digesting the linear RNA molecules; and
(vi) subjecting the at least one circular RNA molecule to reverse transcription rolling circular amplification, obtaining at least one single-stranded cDNA molecule, wherein the at least one single-stranded cDNA molecule carries at least 1, preferably between 2 and 500, copies of the sequence of the at least one RNA molecule;
   wherein the at least one single-stranded cDNA molecule carrying at least 1 copy of the sequence of the at least one RNA molecule is suitable for sequencing, preferably single molecule sequencing. More preferably, the sequencing is performed with the Oxford Nanopore Sequencing platform (nanopore sequencing).

In an embodiment, the biological sample can be selected from eukaryotic (plants, animals, fungi and unicellular organisms as protists), viruses or prokariotic cell lysate, tissue (including blood and biopsies, in vitro and ex-vivo cells), biological fluids (cord-blood plasma, bronchoalveolar lavage, adult blood plasma, parotid saliva, ovarian follicle fluid, serum, amniotic fluid, seminal plasma, urine, bile, submandibular/sublingual saliva, cerebrospinal fluid), 3D cell cultures.

In an embodiment, the at least one RNA molecule bearing a phosphate or a 2',3'-cyclic phosphate group at the 3' end is generated by treating the biological sample with an endoribonuclease, an exoribonuclease, a ribozyme or a toxin able to cleave mRNA, tRNA, snRNA, snoRNA, Y RNA, lncRNA, piRNA, siRNA, viral RNA (from positive-sense RNA viruses, negative-sense RNA viruses, reverse transcribing viruses, and other RNA species produced by viruses) or rRNA.

In an embodiment, the at least one RNA molecule bearing a phosphate or a 2',3'-cyclic phosphate group at the 3' end is physiologically or pathologically present in a biological sample as a consequence of the effect of an endoribonuclease, an exoribonuclease, a ribozyme or a toxin able to cleave mRNA, tRNA, snRNA, snoRNA, Y RNA, lncRNA, piRNA, siRNA, viral RNA (from positive-sense RNA viruses, negative-sense RNA viruses, reverse transcribing viruses, and other RNA species produced by viruses) or rRNA present in the biological sample.

In an embodiment, the endoribonuclease is preferably selected among RNase A; RNase T1; RNase T2; RNaseI; S7 micrococcal nuclease; staphylococcal nuclease; RNAse L; Angiogenin; colicin E5; tRNA-splicing endonuclease (SE2, SEN34); ferredoxin-like Cas6 and ferredoxin-like CasE; IRE1; Poly(U)-specific endoribonuclease (PP11); Las1; RtcA; Type IB topoisomerase; Cue2 endonuclease²⁶ and Cas proteins.

In an embodiment, the exoribonuclease is preferably represented by USB1.

In an embodiment, the ribozyme is preferably selected from hammerhead ribozyme, hairpin ribozyme, hepatitis delta ribozymes, Varkud satellite (VS) ribozyme.

In an embodiment, the toxin is preferably selected from selected from colicin D and colicin E5, alpha-sarcin, zymocin, PaT, MazF, ChpBK, prrC.

In an embodiment, the at least one RNA molecule to be sequenced is single stranded.

In an embodiment the at least one RNA molecule is contained in the biological sample in a concentration comprised between 10 pM and 100 µM, preferably between 1 nM and 10 µM.

In an embodiment, the method comprises a further step (vi) of generation of a complementary cDNA strand of the at least one single-stranded cDNA molecule, obtaining at least one double-stranded cDNA molecule.

In an embodiment, the phosphorylation step (ii) is carried out using a phosphorylating enzyme selected from T4 PNK 3' minus, T4 PNK and recombinant versions of T4 PNK (e.g. Optikinase^{™}).

In an embodiment, the ligation step (iii) is carried out using a first ligase enzyme selected from RtcB, Archease, Arabidopsis Thaliana tRNA ligase, and eukaryotic tRNA ligase.

In an embodiment, the self-ligation step (iv) is carried out using a second ligase enzyme selected from T4 Rnl1, T4 Rnl2, T4 Rnl2tr, T4 Rnl2 K227Q, Mth Rnl, and ATP-independent ligase that catalyzes the intramolecular ligation (e.g. circligase^{™}, circligaseII^{™}).

In an embodiment, the digestion step (v) is carried out with a 5'-3' exoribonuclease or a 3'-5' exoribonuclease, preferably with RNAse R.

In an embodiment, the reverse transcription rolling circular amplification step (vi) is carried out using engineered M MLV-RTs (Moloney Murine Leukemia Virus Reverse Transcriptase) and AMV-RTs (Avian myeoloblastosis virus Reverse Transcriptase), preferably selected from Maxima H minus^{™} Superscript^{™} I-II-III-IV, Sunscript^{™}.

In an embodiment, the generation of the complementary cDNA strand step (vi) is carried out using a DNA polymerase enzyme selected from a Tag Polymerase with 5'-3' exonuclease activity and Gubler-Hoffman method (e.g. Platinum II Taq Hot-Start DNA Polymerase^{™}, AB Tag^{™} PrimeScript^{™}, NEBNext^{®} Ultra^{™} II Non-Directional RNA Second Strand Synthesis).

In a further embodiment the present invention concerns a kit for carrying out the method (disclosed herein) for preparing at least one RNA molecule contained in a biological sample for sequencing, wherein the kit comprises a random RNA linker, and a first ligase enzyme, an exoribonuclease, and optionally a second ligase enzyme, wherein:
(i) the random RNA linker bears -OH group at both ends;
(ii) the ligase enzyme is suitable to ligate the 3' end of an RNA molecule, bearing a phosphate or a 2',3'-cyclic phosphate group at the 3' end and a phosphate group at the 5' end, to the 5' end of the random RNA linker; and
(iii) the exoribonuclease is suitable to enzymatically digest linear RNA molecules; and
(iv) the second ligase enzyme is suitable to circularize a ligation product obtained by ligation of the random RNA linker to the RNA molecule (i.e. ligating the 5' end of the ligation product bearing a phosphate at the 5' end to the 3' end of the ligation product bearing an -OH group at the 3' end).

In an embodiment, the first ligase enzyme is selected from RtcB, Archease, Arabidopsis Thaliana tRNA ligase, and eukaryotic tRNA ligase.

In an embodiment, the second ligase enzyme is selected from T4 Rnl1 T4 Rnl1, T4 Rnl2, T4 Rnl2tr, T4 Rnl2 K227Q, Mth Rnl, and ATP-independent ligase that catalyzes the intramolecular ligation (e.g. circligase^{™}, circligaseII^{™}) .

In an embodiment, the exoribonuclease is RNase R.

In an embodiment, the kit further comprises (a) a phosphorylating enzyme, and/or (b) an endoribonuclease, a ribozyme, or a toxin able to cleave mRNA, tRNA, snRNA, snoRNA, Y RNA, lncRNA, piRNA, siRNA, viral RNA (from positive-sense RNA viruses, negative-sense RNA viruses, reverse transcribing viruses, and other RNA species produced by viruses) or rRNA. Preferably, the kit further comprises an endoribonuclease, wherein the endoribonuclease is RNAseI.

In one or more embodiments, the random RNA linker has a length comprised between 50 and 500 nucleotides.

In one or more embodiments, the random RNA linker has a minimum free energy comprised between -3 and -150 kcal/mol. Preferably, each random RNA linker is preferably designed to have a minimum free energy comprised between of -6 kcal/mol and -24 kcal/mol, with no remarkable secondary structures. Some secondary structures are allowed in the internal portion of the sequence, but not at the 5'/3'-termini. The minimum free energy can be calculated by means of software available to the skilled man.

In one or more embodiments, the random RNA linker has a nucleotide sequence as set forth in SEQ ID No.:3.

The random RNA Linker can be either chemically synthesized or in vitro transcribed and purified, according to the common general knowledge of the expert in the field.

The 5'-OH group of the random linker can be chemically or enzymatically generated. If enzymatically generated, the 5'-OH can be obtained with the catalytic activity of (i) a ribozyme acting in -cis (encoded by *in vitro* transcribed sequence) or in -trans (acting on the *in vitro* transcribed sequence) ii) enzymes leaving a 5'-OH group, as calf intestinal phosphatase, or (iii) a toxin selected from: colicin D and colicin E5, alpha-sarcin, zymocin, Pichia acaciae killer toxin (PaT), MazF, ChpBK, prrC.

The random RNA linker can contain at least one, preferably between 1 and 109 nucleotides modified with at least one of the following modifications: LNA, PNA, 2-Aminopurine, 2,6-Diaminopurine (2-Amino-dA), 6mA, 5-Bromo dU, Inverted dT, 5-Methyl dC, 8-aza-7-deazaguanosine, 5-hydroxybutynl-2'-deoxyuridine, 5-Nitroindole, 2'-O-Methyl A, 2'-O-Methyl G, 2'-O-Methyl C, 2'-O-Methyl U, 2' fluorine A, 2' fluorine C, 2' fluorine G, 2' fluorine U, 2-MethoxyEthoxy A, 2-MethoxyEthoxy MeC, 2-MethoxyEthoxy G, 2-MethoxyEthoxy T, 5-Bromo dU, 2-Aminopurine, inverted dT, 2,6-Diaminopurine, deoxyUridine, inverted Dideoxy-T, 5-Methyl dC, dideoxy-C, deoxylnosine, a universal base comprising 5-Nitroindole, morpholino, a 2'-0-Methyl R A base, isodC, iso-dG, ribonucleotide, a threose nucleotide analogue, a protein nucleotide analogue, a glycoic nucleotide analogue, a locked nucleotide analogue, a chain terminating nucleotide analogue, dihydrouridine, thiouridine, pseudouridine, queuosine, and wyosine phosphorylated ribonucleotide, a modified sugar, an unnatural bond, an abasic site, a dideoxy base, a 5-methyl base, or a spacer selected from Carbon spacer (RNA 5'-0-(CH₂)₃-PO₄-3'RNA), photo-cleavable spacer, RNA 5' O-triethylene glycol-PO₄-3'RNA, 18-atom hexa-ethyleneglycol and 1',2'-dideoxyribose . Preferably, the random RNA linker contains from 1 to 25 modified nucleotides in the first 25 bases from the 5' end and in the last 25 bases from the 3' end.

The inventors developed a library preparation method for nanopore sequencing of short RNA molecules bearing a 3'-P signature, which was validated in the setting of ribosome-profiling (Ribo-seq). In particular, CircAID-p-seq is a highly sensitive RT-RCA-based method that enables detection of low abundance short RNA molecules and is therefore potentially applicable to single-cell technologies.

This method allows to remarkably shorten the time necessary for Ribo-seq library preparation from more than one week, as currently required for the standard protocol³, and significantly reduce the technical steps (dephosphorylation, gel extraction, purification), thus lowering the probability of bias introduction. The present method allows any RNA footprinting study employing enzymatic cleavage leaving 3'-P/cP terminus. Additionally, studies related to cancer and neurodegenerative, autoimmune and infectious disorders, as well as a number of cellular functions where 3'-P/cP-terminated RNA molecules have been reported to be involved, will be particularly advantaged by the instant method. In particular, this method is suitable for the characterization of the endonucleolytic activity of specific enzymes, ribozymes or toxins²⁷, including RNA editing CRISPR-Cas²⁸ systems. Finally, the method herein disclosed allows for fast sequencing pipelines without the need of expensive laboratory equipment, even in resource-limited settings.

### RESULTS

Cellular RNAs can possess a hydroxyl group (-OH), a phosphate group (-P), or a 2',3'-cyclic phosphate group (-cP) at their termini. RNA cleavage by many endoribonucleases often leaves 3'-P or 3'-cP ends, which are not compatible substrates for ATP-dependent ligases (e.g. T4 RNA ligases). A methodologically relevant setting involving the use of endoribonucleases to cleave RNA strands and subsequent ligation events is represented by Ribo-seq for RNA footprinting, which is based on the following steps: (i) cell lysis, (ii) endonuclease (e.g. RNase I) digestion of ssRNAs, (iii) collection of 25-35 nt-long fragments (*bona fide* RPFs), (iv) library preparation, (v) deep sequencing and (vi) final alignment to a reference protein coding transcriptome.

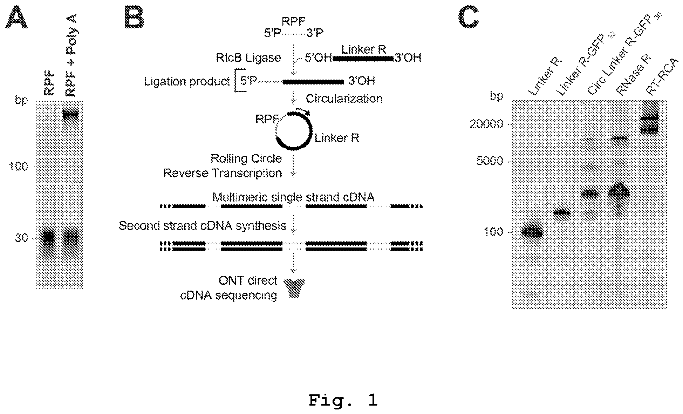

To uncover the fraction of actual RPFs out of the whole population of fragments resulting from RNase I cleavage, the present inventors took advantage of 3' polyadenylation treatment. The results show that around 500 of the 25-35 nt long fragments obtained from cultured cells (MCF7) reacted in the polyadenylation reaction (Figure 1A). This indicates that size-selected RPFs are contaminated with RNA species harboring 3'-OH ends, which can be captured by standard ligation processes yielding a higher background noise.

To overcome the limitations of the currently available Ribo-seq library preparation strategies, the present inventors sought to develop a method allowing (i) the preservation of 3'-P/cP signatures and (ii) independence from PCR amplification steps. To accomplish such goal, the inventors used (i) an enzyme able to ligate 5'-OH to 3'-P/cP termini (RtcB ligase) ^{29,30}, and (ii) a linker suitable for PCR-free nanopore sequencing. In particular, the inventors designed a method dedicated to direct cDNA nanopore sequencing. To provide a proof of concept of the feasibility of this method, the inventors first used a 30 nt-long synthetic RNA fragment bearing -P groups at both 5' and 3' ends (*5'P-GFP-3'P*) as a surrogate of cell-derived and 5' phosphorylated RPFs. The *GFP* fragment has the nucleotide sequence set forth in SEQ ID No.: 1.

In the cirAID-p-Seq approach (Figure 1B), the inventors first ligated the 5'-OH end of a random RNA linker to the 3'-P terminus of the *5'P-GFP-3'P* fragment by means of RtcB ligase. The ligation product was then separated by TBE-urea PAGE, size-selected and gel-purified for the following reaction (Figure 1C). Next, the inventors used T4 RNA ligase I to circularize the *5'P-GFP-linkerR-3'OH* product. In order to confirm the presence of a circular RNA structure, the circularization product was treated with RNase R, an exoribonuclease that digests all linear RNAs instead preserving circular RNAs. After TBE-urea PAGE separation of the RNAse R reaction (Figure 1C), the circularized product (*circGFP-linkerR*) was detected at the expected molecular weight, thus confirming the stability and proper circularization of the construct. The *circGFP-linkerR* product was then gel-purified and subjected to reverse transcription rolling circular amplification (RT-RCA)^{31,32} to obtain a long multimeric single-stranded cDNA molecule (140-15000 nt) carrying many copies of the inserted fragment. As a final checkpoint before sequencing, the RT-RCA product was separated by TBE-urea PAGE, which confirmed the presence of the multimeric cDNA product in the expected size range (Figure 1C).

This method enables the enrichment of 3'-P/cP-endowed RNA fragments because this signature is essential for the efficiency of the overall protocol. This approach is compatible with downstream PCR-free direct cDNA nanopore sequencing and allows multiplexed assays when combined with barcoded linkers.

The library preparation method object of the present disclosure represents the first PCR-free protocol for selective incorporation of 3'-P/cP-terminated RNA fragments and suitable for nanopore sequencing.

### Nanopore sequencing of short 3'-P-terminated RNA fragments

Both GFP-based libraries described above were sequenced with MinION from Oxford Nanopore Technologies (ONT) by using R9.4 flow cells and the 1D chemistry for direct cDNA sequencing.

The inventors performed the synthesis of the second (complementary) cDNA strand, followed by end repair and dA-tailing to suit the ONT protocol for cDNA sequencing. From the sequencing of a library input of 60 ng, the inventors obtained about 1.5 million of "passed" reads (MinKNOWN basecalling) in 2 hours, with an average basecalling quality score of 10.5 and failed reads below 5%.

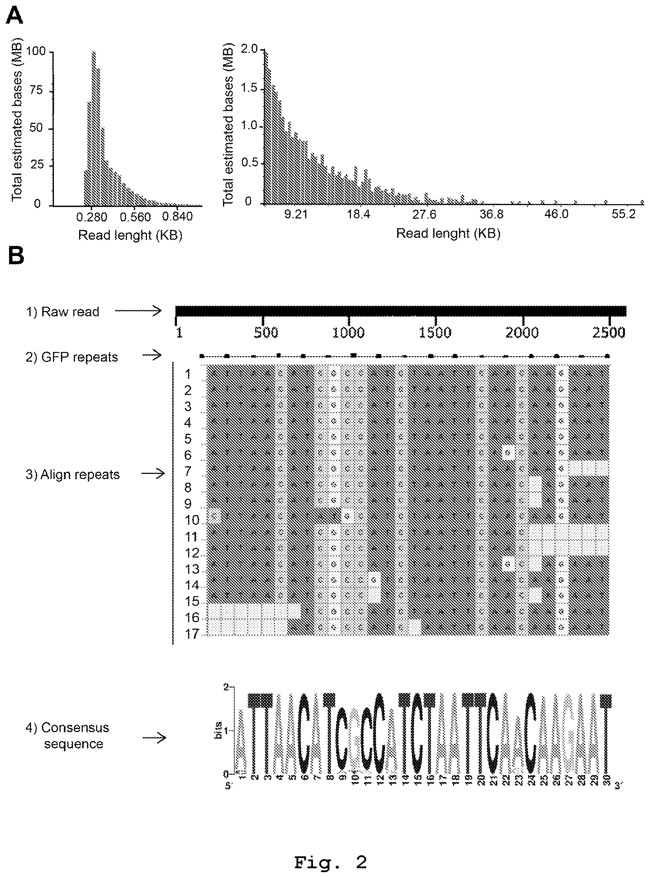

The length distribution of the reads showed a major peak at about 300 nt, with around 10% of the reads spanning a much broader distribution, from 1 KB to more than 50 KB (Figure 2A). This indicates that the RT-RCA reaction exhibits maximum efficiency at 2-3 rounds of reverse transcription, but can generate up to 500 copies of the original template. After BLASTn alignment of the "passed" reads to the reference GFP sequence, 100% of the reads appeared to bear at least one 30-mer GFP fragment. A representative alignment of 17 GFP fragments repeated within a sampled 2.5 KB read highlights the importance of the repeats to computationally generate a consensus sequence, whose accuracy (% identity to original sequence) will be proportional to the number of repeats (Figure 2B), which in turn depends on the efficiency of the RT-RCA reaction.

Overall, the present results provide evidence that the library preparation method (i) can incorporate short synthetic RNA molecules resembling endogenous cleaved RNAs bearing a 3'-P signature, and (ii) is effectively applicable to the ONT sequencing platform.

### circAID-p-seq enables detection of ribosome footprints after RNase I digestion

Since the circAID-p-Seq provided a high sequencing depth, which is required in ribosome profiling experiments, and demonstrated the applicability of this method on the ONT platform with a synthetic 30-mer GFP fragment, the inventors wanted to investigate whether ribosome footprints from GFP-transfected HEK293T cells were identifiable through the present method.

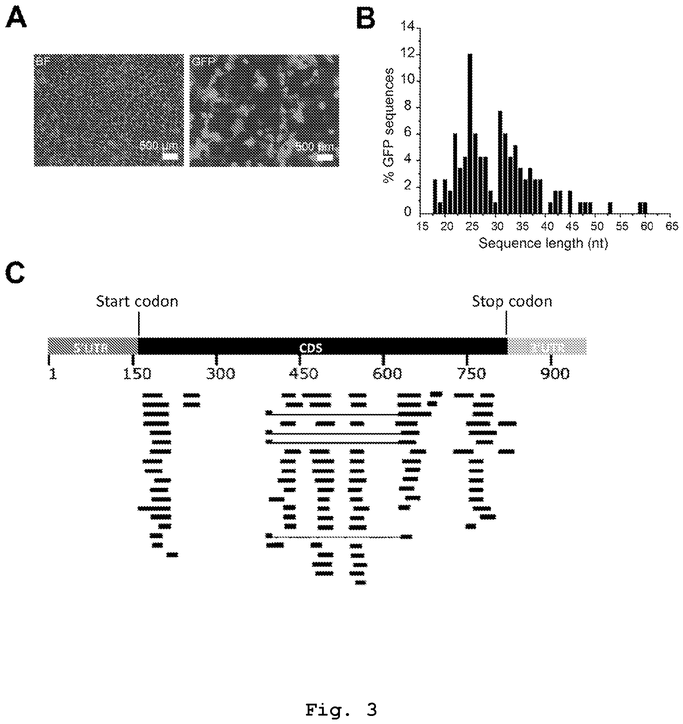

The inventors reasoned that HEK293T transiently transfected with a GFP overexpressing plasmid will have the advantage (i) to ensure a fast identification of RPFs thanks to the orthogonal reference sequence with a well-defined open reading frame and (ii) to produce great amounts of the recombinant protein, with a likely high footprint density on the GFP mRNA. GFP protein appeared to be expressed at high levels after 24 hours from transfection (Figure 3A). Cytoplasmic cell lysates were treated with RNase I to digest all the RNA strands not protected by ribosomes and generate 3'-P-bearing RPFs, which were purified according to Ingolia et al.³³. A total of 450 ng of purified RNA was used as input for library preparation, and the resulting cDNA library was sequenced on MinION for 6 hours. The output generated sequences with a quality score of about 10, with less than 10% failed reads. BLASTn alignment of the MinKNOWN basecalled reads to the reference GFP mRNA allowed the detection of repeated GFP mRNA fragments. The length distribution of GFP fragments ranged between 18 and 60 nt, with an accumulation of reads around 25 and 31 nt (Figure 3B), as expected from a canonical RPF length distribution³⁴. All GFP fragments mapped to the coding sequence, with no coverage of 3' and 5' UTRs (Figure 3C), suggesting that these GFP fragments are authentic RPFs and not footprints deriving from non-ribosomal ribonucleoprotein complexes³⁵.

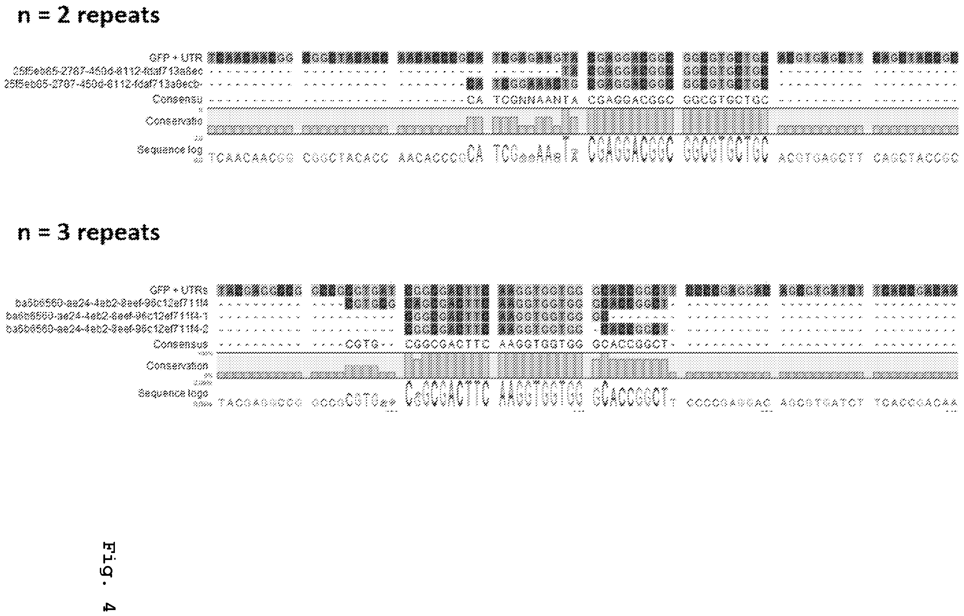

Moreover, the present inventors observed that the resulting consensus sequence achieved excellent accuracy (96.5%) with two repeats, 100% consensus accuracy was obtained with three repeats. This result demonstrates that our strategy of library preparation allows the correct identification of the RNA fragment included in reads containing at least 3 repeats (Table 1; Figure 4).

**Table 1. Average accuracy of the consensus sequence generated by an increasing number of repeats in a single read.**

| **n° of repeats** | **Average accuracy (% identity to expected sequence)** |
|---|---|
| 2 | 96.5% |
| 3 | 100% |
| 4 | 100% |

These results further confirm that CircAID-p-Seq generates ribosome profiling libraries suitable for the ONT platform, and provide evidence that this protocol (CircAID-p-Seq) enables specific detection of actual ribosome footprints along transcripts.

### MATERIALS AND METHODS

### Ribosomal Protected Fragment and linker

Custom linker (having the nucleotide sequence as set forth in SEQ ID No.: 3) was synthesized by IMMAGINA BioTechnology s.r.l (Trento) and consists of 109-mer oligonucleotide with -OH groups at both ends.

Ribosomal protected fragments (RPFs), which consist of 30-mer oligonucleotides having the nucleotide sequence set forth in SEQ ID No.: 1 with 5'-P and 3'-P, were either synthesized by Integrated DNA Technologies (Coralville), or generated in vitro.

In vitro generated RPFs were obtained from HEK293T (Human Embryonic kidney) cells (SIGMA, cat. n°12022001) transfected with a plasmid encoding GFP (pMAX_GFP^{™}, Lonza cat.n° V4XP-3024- SEQ ID No.: 2). Cells were monitored for GFP expression 24 h later by fluorescence microscope (Olimpus DP70). Transfected cells were treated with CHX (10 ug/mL, SIGMA cat. n**°** 01810) for 5 min at 37°C and lysed. RPFs were generated by treating 0.3 AU 260 nm of CHX-treated cellular lysate with 2.25 U of RNAseI (Ambion, cat. n**°** AM2295) in W-buffer (Immagina Biotechnology cat. n**°** #RL001-4) at room temperature for 45 min (as described in Clamer et al., 2018³⁶).

RNAseI digestion was stopped by adding 10U of Superase Inhibitor (Thermo Scientific, cat. n**°** AM2696) for 10 min on ice. After digestion, lysate was purified (as described in Ingolia et al., 2009³³) and treated with 1% SDS (Sigma cat. n**°** 05030) and 0.1 mg Proteinase K (Euroclone, cat. n**°** EMR022001) at 37°C for 75 min. Total RNA was extracted by acid-phenol: chloroform, ph 4.5 (Ambion, cat n**°** AM9722). RNA was precipitated with isopropanol, air-dried, resuspended in 10 mM Tris-HCl pH 8 and analyzed on 15% TBE-urea polyacrylamide gel (Invitrogen, cat n**°** EC6885BOX). 30-mer RPFs were size-selected and extracted from gel (according to Ribolace protocol, Immagina Biotechnology)(Clamer et al. 2018)³⁶.

In-vitro generated RPFs fragments upon purification, were subjected to 5' phosphorylation with T4 PNK 3' minus (NEB, cat n**°** M0236S) before capture with the linker R.

### RPFs fragment-linker ligation

RPFs fragment both phosphorylated at the 5' and 3' ends were used to be ligated with Linker R, (Immagina Biotechnology, cat. n**°** #RLP001-1), by RtcB ligase (NEB, cat. n**°** M0458S), according to the following reaction condition:90 pmol of RPFs, 30 pmol of Linker R, 45 pmol RtcB ligase, Buffer RtcB ligase 1X, 100 µM GTP, 1 mM MnCl₂ in a final volume of 30 µL. The reaction was incubated 2h at 37°C, then the mix was loaded in a 15% acrylamide/8M urea precast gel (Invitrogen, cat n**°** EC6885BOX) and the product of interest (140 nt length) was purified through gel extraction to control the efficiency of the reaction. The gel purification step is not essential to the exploitation of the overall workflow

### Circularization and RNase R treatment

Circularization of RtcB ligated product was carried out at 25 °C for two hours, in a total volume of 20 µL containing 10 U of T4 RNA Ligase 1 (NEB, cat n**°** M0204L), 1X Buffer T4 RNA ligase, 20% PEG8000, 50 µM ATP. The circularization reaction was afterwards incubated at 37 °C for 1h with 20 U of RNase R (Lucigen, cat n°RNR07250), in order to remove all the undesired products (i.e. linear RNA or concatameric product). Circular RNA product was loaded on 15% Acrylamide/ 8M Urea pre-cast gel (Invitrogen, cat n°EC6885BOX) and purified through gel extraction to control the efficiency of the reaction. The gel purification step is not essential to the exploitation of the overall method.

### Reverse Transcription - Rolling Circle Amplification (RT-RCA) and Second Strand synthesis

RT-RCA was performed using the primer, annealing to the 3' region of linker, in 20 µL with Maxima H Minus Reverse Transcriptase (Thermo Fisher, cat n°EP0752) under the following conditions: 50 ng of circular RNA, 200 U of Reverse Transcriptase, 1X Buffer RT, 0.5 mM dNTPs, 50 pmol RT-RCA Rev primer (having the nucleotide sequence set forth in SEQ ID No.: 5), 10% Glycerol. The reaction was carried out at 42°C for 4 hours, then stopped at 70°C for 10 min. After cDNA synthesis, circular RNA template was hydrolyzed by adding 0.1 N NaOH for 10 min at 70°C.

To generate a second strand from single strand cDNA molecules, one cycle of PCR was performed with Super AB Tag Polymerase (AB Analitica cat n°06-36-020) using the RT-RCA Fw primer, annealing to the 5' region of linker, having the nucleotide sequences set forth in SEQ ID No.: 4. The reaction included 20 ul from the RT reaction, 1x Buffer, 0.2 mM dNTPs, 2 mM MgCl₂, 1.25U Taq Polymerase, 50 pmol RT-RCA Fw primer in a total volume of 50 ul and subjected to the following program: initial denaturation at 95°C, one cycle of 95°C for 30 sec, 51°C for 30 sec and 70°C for 2 minutes. Double strand cDNA was purified using AMPure XP beads (Agencourt, cat n°A63881) according to manufacturer's instructions.

### Library preparation and Nanopore sequencing

Purified cDNA was prepared for nanopore sequencing. Briefly, cDNA was subjected to end repair and dA-tailing reaction using NEBNext End repair/dA-tailing module (NEB, cat. n°E7546S) following the manufacturer's instruction and incubated 5 min at 20°C, 5 min at 65°C. The reaction mix was purified with AMPure XP beads (Agencourt). ONT Adapter mix was added according to direct cDNA sequencing kit protocol (SQK-DCS109, ONT), then loaded on a R9.4 flow cell and sequenced with MinION sequencer.

### Data analysis

In the bioinformatic analysis for direct cDNA sequencing, all the alignment against a reference sequence was performed with BLAST-n or CLC Genomics Workbench (QIAGEN). For the consensus sequence generation Mesquite software was used to align single GFP repeats and WebLogo online tool for the final consensus sequence generation.

### Schematic description of the circAID-p-seq method

### Step 1. RPFs Phosphorylation.

Upon selection and purification, RPFs bearing a 3'P or 3'cP will be subjected to 5' phosphorylation by T4 PNK 3' Minus, according to the protocol indicated in Table 2.

**Table 2.**

| **Component** | **Amount Range** | **Preferred amount** |
|---|---|---|
| 10x Buffer | n.d. | 1X |
| 10 mM ATP, alternatively 10 mM dATP | 0.05-2 mM | 1 mM |
| 10 U/ µL T4 PNK 3' minus | 1-20 U | 10 U |
| RPFs | 10-300 pmol | 90 pmol |
| H₂O | n.d. | Up to 50 µL |

| | | |
|---|---|---|
| n.d., not defined | | |

The reaction is incubated 1 hour at 37 °C, then purified trough Zymo column purification kit.

### Step 2. RtcB ligation.

RPFs from step 1, both phosphorylated at 5' and 3' termini, will be ligated to a 109 nt linker RNA molecule (Linker R), *via* RtcB ligase. RtcB ligase will join 5'OH termini of linker R to a 3'P/3'cP termini of RPFs, according to the protocol indicated in Table 3.

**Table 3.**

| **Component** | **Amount Range** | **Preferred amount** |
|---|---|---|
| 10x Buffer | n.d. | 1 X |
| 1mM GTP | n.d. | 0.1 mM |
| 10 mM MnCl₂ | n.d. | 1 mM |
| 10 U/µL RtcB ligase | 5-30 U | 30 U |
| Linker R | 5-30 pmol | 30 pmol |
| RPFs | 15-90 pmol | 90 pmol |
| H₂O | n.d. | Up to 30 µL |

### Incubate 2 hours at 37 °C.

The reaction is loaded on 15% Tris-borate-EDTA (TBE)-urea acryl-amide gels and ligation product is size selected, gel extracted and precipitated in isopropanol, finally resuspended in 8 µL of water. The purified product (RPFs-Linker R) is around 140 nt length, bearing a 5'P and 3' OH termini. Such a step is not essential for performing the method disclosed herein.

### Step 3. Circularization 5'P-RPFs-Linker R-3'OH product

The 5'P-RPFs-Linker R-3'OH product is subjected to circularization trough the ligation of 5'P termini and 3'OH termini by T4 RNA ligase 1. Reaction condition are indicated in Table 4.

**Table 4.**

| **Component** | **Amount Range** | **Preferred amount** |
|---|---|---|
| 10x Buffer | n.d. | 1 X |
| 1mM ATP | 0,05-2 mM | 0.05 mM |
| 50% PEG800 | 10%-25% | 20 % |
| 10 U /µL T4 RNA Ligase | n.d. | 10 U |
| 5'P/RPFs-Linker R/3'OH | n.d. | 8 µL |

| | | |
|---|---|---|
| Incubation: 2h at 25 °C. | | |

### Step 4. RNase R

The reaction conditions are provided in Table 5.

**Table 5.**

| **Component** | **Amount Range** | **Preferred amount** |
|---|---|---|
| 10x Buffer | n.d. | 1X |
| 20 U /µL RNase R | n.d. | 20 U |
| Reaction mix from step 3 | n.d. | 20 µL |
| H₂O | n.d. | up to 25 µL |

### Incubate 1 h at 37 °C.

The reaction is loaded on 15% Tris-borate-EDTA (TBE)-urea acryl-amide gels and circular RNA molecule is gel extracted (the gel extraction is not an essential step for performing the method disclosed herein). After isopropanol precipitation, circular RNA is resuspended in 8 µL and quantified (QuBit quantification).

### Step 5. Reverse Transcription - Rolling Circle Amplification (RT-RCA).

For the generation of multimeric single strand cDNA the reagents are mixed in the amounts indicated in Table 6.

**Table 6.**

| **Component** | **Amount Range** | **Preferred amount** |
|---|---|---|
| 10 mM dNTPs | 0,1mM - 1 mM | 0.5 mM |
| Circular RNA (from step 4) | 0,02 - 300 pmol | 1 pmol |
| 20 µM RT-RCA Rev primer | 0.5 µM - 5 µM | 2.5 µM |
| H₂O | n.d. | Up to 13 µL |

Heat the circular RNA-primer mix at 65°C for 5 minutes, and then incubate on ice for at least 1 minute. Add to the annealed RNA the reagents in the amounts indicated in Table 7.

**Table 7.**

| **Component** | **Amount Range** | **Preferred amount** |
|---|---|---|
| 10x Buffer | 0, 05x - 2x | 1X |
| 100% glycerol | 0%-25% | 10% |
| 200 U/ µL Maxima H minus RT | 50U - 200 U | 200 U |

Incubate 4 h at 42 °C, then add 0.1N NaOH and heat the mix for 20 min at 70 °C. Finally, precipitate the reaction, adding: 156 µL of nuclease free water, 20 µL sodium acetate (3M), 300 µL isopropanol and 2 µL of Glycoblue. After precipitation, resuspend in 20 µL of nuclease free water.

### Step 6. Second strand synthesis.

To generate a second strand from the single strand cDNA molecules (generated in step 5), one cycle of PCR is performed under the reaction conditions provided in Tables 8 and 9.

**Table 8.**

| **Component** | **Amount Range** | **Preferred amount** |
|---|---|---|
| 10x Buffer | 0.05x - 1.5x | 1 X |
| 10 mM dNTPs | 0.02 mM - 1 mM | 0.2 mM |
| 100 mM MgCl₂ | 0.5 mM - 3 mM | 2 mM |
| 20 µM RT-RCA Fw primer | 0.1 µM - 2 µM | 1 µM |
| cDNA (from step 5) | n.d. | 20 µL |
| 5U/µL Super AB Tag | 0.5U - 2U - | 1.5 U |
| Nuclease free water | n.d | Up to 50 µL |

**Table 9.**

| **Step** | **Temperature** | **Time** |
|---|---|---|
| Initial denaturation | 95°C | 3 min |
| 1 Cycle | 95°C | 30 sees |
| | 51°C | 30secs |
| | 72°C | 2 min |
| Hold | 4°C | |

The reaction is purified by adding 45pL of AMPure XP beads (agencourt). Final product is eluted in a total volume of 25 µL of nuclease free water.

### Step 7. ONT Library preparation.

Purified double strand cDNA (see step 6) is used for the ONT library preparation, following the protocol direct-cDNA sequencing kit (SQK-DCS109), starting from "End Prep Step".

### References

(1) Lee, F. C. Y.; Ule, J. Advances in CLIP Technologies for Studies of Protein-RNA Interactions. Mol. Cell 2018, 69 (3), 354-369.
(2) Huang, R.; Han, M.; Meng, L.; Chen, X. Transcriptome-Wide Discovery of Coding and Noncoding RNA-Binding Proteins. Proc. Natl. Acad. Sci. 2018, 115 (17), E3879-E3887.
(3) Ingolia, N. T.; Ghaemmaghami, S.; Newman, J. R. S.; Weissman, J. S. Genome-Wide Analysis in Vivo of Translation with Nucleotide Resolution Using Ribosome Profiling. Science 2009, 324 (5924), 218-223.
(4) Ramanathan, M.; Porter, D. F.; Khavari, P. A. Methods to Study RNA-Protein Interactions. Nat. Methods 2019, 16 (3), 225-234.
(5) Ramanathan, M.; Majzoub, K.; Rao, D. S.; Neela, P. H.; Zarnegar, B. J.; Mondal, S.; Roth, J. G.; Gai, H.; Kovalski, J. R.; Siprashvili, Z.; et al. RNA-Protein Interaction Detection in Living Cells. Nat. Methods 2018, 15 (3), 207-212.
(6) Metzker, M. L. Sequencing Technologies - the next Generation. Nat. Rev. Genet. 2010, 11 (1), 31-46.
(7) Ozsolak, F.; Milos, P. M. RNA Sequencing: Advances, Challenges and Opportunities. Nat. Rev. Genet. 2011, 12 (2), 87-98.
(8) Thiyagarajan, N.; Ferguson, R.; Subramanian, V.; Acharya, K. R. Structural and Molecular Insights into the Mechanism of Action of Human Angiogenin-ALS Variants in Neurons. Nat. Commun. 2012, 3 (1), 1121.
(9) Sheng, J.; Xu, Z. Three Decades of Research on Angiogenin: A Review and Perspective. Acta Biochim. Biophys. Sin. (Shanghai). 2016, 48 (5), 399-410.
(10) Maurel, M.; Chevet, E.; Tavernier, J.; Gerlo, S. Getting RIDD of RNA: IRE1 in Cell Fate Regulation. Trends Biochem. Sci. 2019, 39 (5), 245-254.
(11) Yoshinari, S.; Liu, Y.; Gollnick, P.; Ho, C. K. Cleavage of 3'-Terminal Adenosine by Archaeal ATP-Dependent RNA Ligase. Sci. Rep. 2017, 7 (1), 11662.
(12) Filipowicz, W.; Shatkin, A. J. Origin of Splice Junction Phosphate in TRNAs Processed by HeLa Cell Extract. Cell 1983, 32 (2), 547-557.
(13) Shinya, S.; Kadokura, H.; Imagawa, Y.; Inoue, M.; Yanagitani, K.; Kohno, K. Reconstitution and Characterization of the Unconventional Splicing of XBP1u MRNA in Vitro. Nucleic Acids Res. 2011, 39 (12), 5245-5254.
(14) Bradshaw, W. J.; Rehman, S.; Pham, T. T. K.; Thiyagarajan, N.; Lee, R. L.; Subramanian, V.; Acharya, K. R. Structural Insights into Human Angiogenin Variants Implicated in Parkinson's Disease and Amyotrophic Lateral Sclerosis. Sci. Rep. 2017, 7 (1), 41996.
(15) Lu, L.; Li, J.; Moussaoui, M.; Boix, E. Immune Modulation by Human Secreted RNases at the Extracellular Space. Front. Immunol. 2018, 9.
(16) Honda, S.; Morichika, K.; Kirino, Y. Selective Amplification and Sequencing of Cyclic Phosphate-Containing RNAs by the CP-RNA-Seq Method. Nat. Protoc. 2016, 11 (3), 476-489.
(17) Honda, S.; Kirino, Y. Dumbbell-PCR: A Method to Quantify Specific Small RNA Variants with a Single Nucleotide Resolution at Terminal Sequences. Nucleic Acids Res. 2015, 43 (12), e77-e77.
(18) Chen, W.; Moore, J.; Ozadam, H.; Shulha, H. P.; Rhind, N.; Weng, Z.; Moore, M. J. Transcriptome-Wide Interrogation of the Functional Intronome by Spliceosome Profiling. Cell 2018, 173 (4), 1031-1044.e13.
(19) McPike, M. P.; Goodisman, J.; Dabrowiak, J. C. Drug-RNA Footprinting; 2001; pp 431-449.
(20) Wu, C. C.-C.; Zinshteyn, B.; Wehner, K. A.; Green, R. High-Resolution Ribosome Profiling Defines Discrete Ribosome Elongation States and Translational Regulation during Cellular Stress. Mol. Cell 2019, 73 (5), 959-970.e5.
(21) Ingolia, N. T.; Brar, G. A.; Rouskin, S.; McGeachy, A. M.; Weissman, J. S. The Ribosome Profiling Strategy for Monitoring Translation in Vivo by Deep Sequencing of Ribosome-Protected MRNA Fragments. Nat. Protoc. 2012, 7 (8), 1534-1550.
(22) Zhu, Y. Y.; Machleder, E. M.; Chenchik, A.; Li, R.; Siebert, P. D. Reverse Transcriptase Template Switching: A SMART™ Approach for Full-Length CDNA Library Construction. Biotechniques 2001, 30 (4), 892-897.
(23) Balatti, V.; Nigita, G.; Veneziano, D.; Drusco, A.; Stein, G. S.; Messier, T. L.; Farina, N. H.; Lian, J. B.; Tomasello, L.; Liu, C.; et al. TsRNA Signatures in Cancer. Proc. Natl. Acad. Sci. 2017, 114 (30), 8071-8076.
(24) Godoy, P. M.; Bhakta, N. R.; Barczak, A. J.; Cakmak, H.; Fisher, S.; MacKenzie, T. C.; Patel, T.; Price, R. W.; Smith, J. F.; Woodruff, P. G.; et al. Large Differences in Small RNA Composition Between Human Biofluids. Cell Rep. 2018, 25 (5), 1346-1358.
(25) Shigematsu, M.; Kawamura, T.; Kirino, Y. Generation of 2',3'-Cyclic Phosphate-Containing RNAs as a Hidden Layer of the Transcriptome. Front. Genet. 2018, 9.
(26) D'Orazio, K. N.; Wu, C. C.-C.; Sinha, N.; Loll-Krippleber, R.; Brown, G. W.; Green, R. The Endonuclease Cue2 Cleaves MRNAs at Stalled Ribosomes during No Go Decay. Elife 2019, 8*.*
(27) Zhang, Y.; Zhang, J.; Hara, H.; Kato, I.; Inouye, M. Insights into the MRNA Cleavage Mechanism by MazF, an MRNA Interferase. J. Biol. Chem. 2005, 280 (5), 3143-3150.
(28) Tang, Y.; Fu, Y. Class 2 CRISPR/Cas: An Expanding Biotechnology Toolbox for and beyond Genome Editing. Cell Biosci. 2018, 8 (1), 59.
(29) Englert, M.; Sheppard, K.; Aslanian, A.; Yates, J. R.; Soil, D. Archaeal 3'-Phosphate RNA Splicing Ligase Characterization Identifies the Missing Component in TRNA Maturation. Proc. Natl. Acad. Sci. 2011, 108 (4), 1290-1295.
(30) Tanaka, N.; Shuman, S. RtcB Is the RNA Ligase Component of an Escherichia Coli RNA Repair Operon. J. Biol. Chem. 2011, 286 (10), 7727-7731.
(31) Kumar, P.; Johnston, B. H.; Kazakov, S. A. MiR-ID: A Novel, Circularization-Based Platform for Detection of MicroRNAs. RNA 2011, 17 (2), 365-380.
(32) You, X.; Vlatkovic, I.; Babic, A.; Will, T.; Epstein, I.; Tushev, G.; Akbalik, G.; Wang, M.; Glock, C.; Quedenau, C.; et al. Neural Circular RNAs Are Derived from Synaptic Genes and Regulated by Development and Plasticity. Nat. Neurosci. 2015, 18 (4), 603-610.
(33) Ingolia, N. T.; Ghaemmaghami, S.; Newman, J. R. S.; Weissman, J. S. Genome-Wide Analysis in Vivo of Translation with Nucleotide Resolution Using Ribosome Profiling. Science (80-. ). 2009, 324 (5924), 218-223.
(34) Lareau, L. F.; Hite, D. H.; Hogan, G. J.; Brown, P. O. Distinct Stages of the Translation Elongation Cycle Revealed by Sequencing Ribosome-Protected MRNA Fragments. Elife 2014, 3*.*
(35) Ingolia, N. T.; Brar, G. A.; Stern-Ginossar, N.; Harris, M. S.; Talhouarne, G. J. S.; Jackson, S. E.; Wills, M. R.; Weissman, J. S. Ribosome Profiling Reveals Pervasive Translation Outside of Annotated Protein-Coding Genes. Cell Rep. 2014, 8 (5), 1365-1379.
(36) Clamer, M.; Tebaldi, T.; Lauria, F.; Bernabò, P.; Gómez-Biagi, R. F.; Marchioretto, M.; Kandala, D. T.; Minati, L.; Perenthaler, E.; Gubert, D.; et al. Active Ribosome Profiling with RiboLace. Cell Rep. 2018, 25 (4), 1097-1108.e5.

## Claims

1. An in vitro method for preparing at least one RNA molecule, contained in a biological sample, for sequencing comprising the following steps:
(i) obtaining a biological sample comprising at least one RNA molecule, wherein the at least one RNA molecule bears a phosphate or a 2',3'-cyclic phosphate group at the 3' end;
(ii) phosphorylating the at least one RNA molecule at the 5' end, thus introducing a phosphate group at the 5' end of the at least one RNA molecule, and obtaining at least one RNA molecule phosphorylated at both ends;
(iii) ligating the 3' end of the at least one phosphorylated RNA molecule to the 5' end of a random RNA linker, wherein the random RNA linker bears a -OH group at both ends, obtaining at least one first ligation product;
(iv) self-ligating the at least one first ligation product to form at least one circular RNA molecule, wherein the at least one circular RNA molecule is mixed with linear RNA molecules;
(v) digesting the linear RNA molecules;
(vi) subjecting the at least one circular RNA molecule to reverse transcription rolling circular amplification, obtaining at least one single-stranded cDNA molecule, wherein the at least one single-stranded cDNA molecule carries at least 1, preferably between 2 and 500, copies of the sequence of the at least one RNA molecule;
wherein the at least one single-stranded cDNA molecule carrying at least 1 copy of the sequence of the at least one RNA molecule is suitable for sequencing, preferably single molecule sequencing.

2. The method according to claim 1, wherein the method comprises a further step (vii) of generation of a complementary cDNA strand of the at least one single-stranded cDNA molecule, obtaining at least one double-stranded cDNA molecule.

3. The method according to claim 1 to 2, wherein the phosphorylation step (ii) is carried out using a phosphorylating enzyme selected from T4 PNK 3' minus, T4 PNK and recombinant versions of T4 PNK.

4. The method according to anyone of the preceding claims, wherein the ligation step (iii) is carried out using a first ligase enzyme selected from RtcB, Archease, Arabidopsis Thaliana tRNA ligase, and eukaryotic tRNA ligase.

5. The method according to anyone of the preceding claims, wherein the self-ligation step (iv) is carried out using a second ligase enzyme selected from T4 Rnl1, T4 Rnl2, T4 Rnl2tr, T4 Rnl2 K227Q, Mth Rnl and ATP-independent ligase that catalyzes the intramolecular ligation.

6. The method according to anyone of the preceding claims, wherein the digestion step (v) is carried out using a 3'-5' exoribonuclease or a 5'-3' exoribonuclease.

7. The method according to anyone of the preceding claims, wherein the reverse transcription rolling circular amplification step (vi) is carried out using a reverse transcription enzyme selected from engineered M MLV-RTs (Moloney Murine Leukemia Virus Reverse Transcriptase) and AMV-RTs (Avian myeoloblastosis virus Reverse Transcriptase).

8. The method according to anyone of claims 2 to 6, wherein the generation of the complementary cDNA strand step (vii) is carried out using a Taq Polymerase with 5'-3' exonuclease activity.

9. The method according to anyone of the preceding claims, wherein the random RNA linker has a length comprised between 50 and 500 nucleotides.

10. The method according to anyone of the preceding claims, wherein the random RNA linker has a minimum free energy comprised between -3 and -150 kcal/mol.

11. The method according to anyone of the preceding claims, wherein the at least one RNA molecule bearing a phosphate or a 2',3'-cyclic phosphate group at the 3' end is physiologically or pathologically present in the biological sample or is generated by treating the biological sample with an endoribonuclease, an exoribonuclease, a ribozyme or a toxin able to cleave mRNA, tRNA, snRNA, snoRNA, Y RNA, lncRNA, piRNA, siRNA, viral RNA or rRNA.

12. A kit comprising a random RNA linker, and a first ligase enzyme, an exoribonuclease, and optionally a second ligase enzyme, wherein:
(i) the random RNA linker bears -OH group at both ends;
(ii) the ligase enzyme is suitable to ligate the 3' end of an RNA molecule, bearing a phosphate or a 2',3'-cyclic phosphate group at the 3' end and a phosphate group at the 5' end, to the 5' end of the random RNA linker;
(iii) the exoribonuclease is suitable to enzymatically digest linear RNA molecules; and
(iv) the second ligase enzyme is suitable to circularize a ligation product obtained by ligation of the random RNA linker to the RNA molecule.

13. The kit according to claim 12, wherein the first ligase enzyme is selected from RtcB, Archease, Arabidopsis Thaliana tRNA ligase, and eukaryotic tRNA ligase.

14. The kit according to claim 12 or claim 13, wherein the second ligase enzyme is selected from T4 Rnl1, T4 Rnl2, T4 Rnl2tr, T4 Rnl2 K227Q, Mth Rnl and ATP-independent, ligase that catalyzes the intramolecular ligation.

15. The kit according to anyone of claims 12 to 14, wherein the random RNA linker has a length comprised between 50 and 500 nucleotides.

16. The kit according to anyone of claims 12 to 15, wherein the random RNA linker has a minimum free energy comprised between -3 and -150 kcal/mol.

17. The kit according to anyone of claims 12 to 16, further comprising (i) a phosphorylating enzyme, and/or (ii) an endoribonuclease, a ribozyme, or a toxin able to cleave mRNA, tRNA, snRNA, snoRNA, Y RNA, lncRNA, piRNA, siRNA, viral RNA or rRNA.

18. The kit according to anyone of claims 12 to 17, wherein the random RNA linker has a nucleotide sequence as set forth in SEQ ID No.:3.

## Patentansprüche

1. In-vitro-Verfahren zur Bereitstellung mindestens eines RNA-Moleküls, welches in einer biologischen Probe enthalten ist, um dieses zu sequenzieren, wobei es die folgenden Schritte umfasst:
(i) Gewinnen einer biologischen Probe, die mindestens ein RNA-Molekül umfasst, wobei das mindestens eine RNA-Molekül am 3'-Ende mit einem Phosphat oder einer zyklischen 2',3'-Phosphatgruppe versehen ist;
(ii) Phosphorylieren des mindestens einen RNA-Moleküls am 5'-Ende, um auf diese Weise eine Phosphatgruppe am 5'-Ende des mindestens einen RNA-Moleküls einzuführen und mindestens ein RNA-Molekül zu erhalten, das an beiden Enden phosphoryliert ist;
(iii) Ligieren des 3'-Endes des mindestens einen phosphorylierten RNA-Moleküls an das 5'-Ende eines beliebigen RNA-Linkers, wobei der beliebige RNA-Linker an beiden Enden mit einer OH-Gruppe versehen ist, wobei mindestens ein erstes Ligationsprodukt erhalten wird;
(iv) Selbstligieren des mindestens einen ersten Ligationsprodukts, um mindestens ein ringförmiges RNA-Molekül zu bilden, wobei das mindestens eine ringförmige RNA-Molekül mit geradkettigen RNA-Molekülen vermischt ist;
(v) Verdau der geradkettigen RNA-Moleküle;
(vi) Einwirkenlassen von Rolling-Circle-Vervielfältigung mit reverser Transkription auf das mindestens eine ringförmige RNA-Molekül, um auf diese Weise mindestens ein Einzelstrang-cDNA-Molekül zu erhalten, wobei das mindestens eine Einzelstrang-cDNA-Molekül mit mindestens 1, vorzugsweise mit 2 bis 500, Kopien der Sequenz des mindestens einen RNA-Moleküls versehen ist;
wobei das mindestens eine Einzelstrang-cDNA-Molekül, welches mit mindestens 1 Kopie der Sequenz des mindestens einen RNA-Moleküls versehen ist, sich zur Sequenzierung eignet, vorzugsweise zur Einzelmolekülsequenzierung.

2. Verfahren gemäß Anspruch 1, wobei das Verfahren einen weiteren Schritt (vii) des Erzeugens eines komplementären cDNA-Strangs des mindestens einen Einzelstrang-cDNA-Moleküls umfasst, wobei mindestens ein DoppelstrangcDNA-Molekül erhalten wird.

3. Verfahren gemäß Anspruch 1 bis 2, wobei der Phosphorylierungsschritt (ii) unter Verwendung eines Phosphorylierungsenzyms durchgeführt wird, welches aus T4 PNK 3' minus, T4 PNK und rekombinanten Versionen von T4 PNK ausgewählt ist.

4. Verfahren gemäß einem beliebigen der vorhergehenden Ansprüche, wobei der Ligationsschritt (iii) unter Verwendung eines ersten Ligase-Enzyms durchgeführt wird, das aus RtcB, Archease, Arabidopsis-Thaliana-tRNA-Ligase und eukaryotischer tRNA-Ligase ausgewählt ist.

5. Verfahren gemäß einem beliebigen der vorhergehenden Ansprüche, wobei der Selbstligationsschritt (iv) unter Verwendung eines zweiten Ligase-Enzyms durchgeführt wird, das aus T4 Rnl1, T4 Rnl2, T4 Rnl2tr, T4 Rnl2 K227Q, Mth Rnl und ATP-unabhängiger Ligase, welche die intramolekulare Ligation katalysiert, ausgewählt ist.

6. Verfahren gemäß einem beliebigen der vorhergehenden Ansprüche, wobei der Verdauschritt (v) unter Verwendung einer 3'-5'-Exoribonuclease oder einer 5'-3'-Exoribonuclease durchgeführt wird.

7. Verfahren gemäß einem beliebigen der vorhergehenden Ansprüche, wobei der Schritt (vi) der Rollung-Circle-Vervielfältigung mit reverser Transkription unter Verwendung eines reversen Transkriptase-Enzyms durchgeführt wird, das aus künstlich bearbeiteten M MLV-RTs (Moloney Murine Leukemia Virus Reverse Transcriptase) und AMV-RTs (Avian myeoloblastosis virus Reverse Transcriptase) ausgewählt ist.

8. Verfahren gemäß einem beliebigen der Ansprüche 2 bis 6, wobei der Schritt (vii) der Erzeugung des komplementären cDNA-Strangs unter Verwendung einer Taq-Polymerase mit 5'-3'-Exonuclease-Aktivität durchgeführt wird.

9. Verfahren gemäß einem beliebigen der vorhergehenden Ansprüche, wobei der beliebige RNA-Linker eine Länge im Bereich von 50 bis 500 Nukleotiden hat.

10. Verfahren gemäß einem beliebigen der vorhergehenden Ansprüche, wobei der beliebige RNA-Linker einen Mindestwert an freier Energie im Bereich von -3 bis -150 kcal/mol hat.

11. Verfahren gemäß einem beliebigen der vorhergehenden Ansprüche, wobei das mindestens eine RNA-Molekül, welches am 3'-Ende mit einem Phosphat oder einer zyklischen 2',3'-Phosphatgruppe versehen ist, auf physiologische oder pathologische Weise in der biologischen Probe vorliegt oder dadurch entsteht, dass die biologische Probe mit einer Endoribonuclease, einer Exoribonuclease, einem Ribozym oder einem Toxin behandelt wird, welche(s) dazu befähigt ist, mRNA, tRNA, snRNA, snoRNA, Y RNA, lncRNA, piRNA, siRNA, virale RNA oder rRNA zu spalten.

12. Kit, das einen beliebigen RNA-Linker sowie ein erstes Ligase-Enzym, eine Exoribonuclease und möglicherweise ein zweites Ligase-Enzym umfasst, wobei:
(i) der beliebige RNA-Linker an beiden Enden mit einer OH-Gruppe versehen ist;
(ii) das Ligase-Enzym dafür geeignet ist, das 3'-Ende eines RNA-Moleküls, welches am 3'-Ende mit einem Phosphat oder einer zyklischen 2',3'-Phosphatgruppe versehen ist und am 5'-Ende mit einer Phosphatgruppe versehen ist, an das 5'-Ende des beliebigen RNA-Linkers zu ligieren;
(iii) die Exoribonuclease dafür geeignet ist, geradkettige RNA-Moleküle enzymatisch zu verdauen; und
(iv) das zweite Ligase-Enzym dafür geeignet ist, den Ringschluss eines Ligationsprodukts zu bewirken, wie es erhalten wurde, indem der beliebige RNA-Linker an das RNA-Molekül ligiert wird.

13. Kit gemäß Anspruch 12, wobei das erste Ligase-Enzym aus RtcB, Archease, Arabidopsis-Thaliana-tRNA-Ligase und eukaryotischer tRNA-Ligase ausgewählt ist.

14. Kit gemäß Anspruch 12 oder Anspruch 13, wobei das zweite Ligase-Enzym aus T4, Rnl1, T4 Rnl2, T4 Rnl2tr, T4 Rnl2 K227Q, Mth Rnl und ATP-unabhängiger Ligase, welche die intramolekulare Ligation katalysiert, ausgewählt ist.

15. Kit gemäß einem beliebigen der Ansprüche 12 bis 14, wobei der beliebige RNA-Linker eine Länge im Bereich von 50 bis 500 Nukleotiden aufweist.

16. Kit gemäß einem beliebigen der Ansprüche 12 bis 15, wobei der beliebige RNA-Linker einen Mindestwert an freier Energie im Bereich von -3 bis -150 kcal/mol hat.

17. Kit gemäß einem beliebigen der Ansprüche 12 bis 16, wobei es weiterhin (i) ein phosphorylierendes Enzym und/oder (ii) eine Endoribonuclease, ein Ribozym oder ein Toxin umfasst, welche(s) dazu befähigt ist, mRNA, tRNA, snRNA, snoRNA, Y RNA, lncRNA, piRNA, siRNA, virale RNA oder rRNA zu spalten.

18. Kit gemäß einem beliebigen der Ansprüche 12 bis 17, wobei der beliebige RNA-Linker eine Nukleotidsequenz gemäß den Angaben in SEQ ID Nr.: 3 aufweist.

## Revendications

1. Procédé *in vitro* pour la préparation d'au moins une molécule d'ARN, contenue dans un échantillon biologique, pour un séquençage comprenant les étapes suivantes :
(i) obtention d'un échantillon biologique comprenant au moins une molécule d'ARN, l'au moins une molécule d'ARN portant un groupe phosphate ou un groupe phosphate 2',3'-cyclique à l'extrémité 3' ;
(ii) phosphorylation de l'au moins une molécule d'ARN à l'extrémité 5', introduisant ainsi un groupe phosphate à l'extrémité 5' de l'au moins une molécule d'ARN, et obtention d'au moins une molécule d'ARN phosphorylée aux deux extrémités ;
(iii) ligature de l'extrémité 3' de l'au moins une molécule d'ARN phosphorylée à l'extrémité 5' d'un lieur d'ARN aléatoire, le lieur d'ARN aléatoire portant un groupe -OH aux deux extrémités, obtenant au moins un premier produit de ligature ;
(iv) auto-ligature de l'au moins un premier produit de ligature pour former au moins une molécule d'ARN circulaire, l'au moins une molécule d'ARN circulaire étant mélangée avec des molécules d'ARN linéaires ;
(v) digestion des molécules d'ARN linéaires ;
(vi) soumission de l'au moins une molécule d'ARN circulaire à une amplification circulaire roulante de transcription inverse, pour obtenir au moins une molécule d'ADNc simple brin, l'au moins une molécule d'ADNc simple brin portant au moins 1, préférablement entre 2 et 500, copies de la séquence de l'au moins une molécule d'ARN ; l'au moins une molécule d'ADNc simple brin portant au moins 1 copie de la séquence de l'au moins une molécule d'ARN étant appropriée pour un séquençage, préférablement un séquençage d'une seule molécule.

2. Procédé selon la revendication 1, dans lequel le procédé comprend une étape supplémentaire (vii) de génération d'un brin d'ADNc complémentaire de l'au moins une molécule d'ADNc simple brin, obtenant au moins une molécule d'ADNc double brin.

3. Procédé selon la revendication 1 à 2, dans lequel l'étape de phosphorylation (ii) est réalisée en utilisant une enzyme phosphorylante choisie parmi T4 PNK 3' moins, T4 PNK et des versions recombinantes de T4 PNK.

4. Procédé selon l'une quelconque des revendications précédentes, dans lequel l'étape de ligature (iii) est réalisée en utilisant une première enzyme ligase choisie parmi RtcB, Archéase, une ligase d'ARNt d'Arabidopsis Thaliana et une ligase d'ARNt d'eucaryote.

5. Procédé selon l'une quelconque des revendications précédentes, dans lequel l'étape d'auto-ligature (iv) est réalisée en utilisant une deuxième enzyme ligase choisie parmi T4 Rnl1, T4 Rnl2, T4 Rnl2tr, T4 Rnl2 K227Q, Mth Rnl et une ligase indépendante de l'ATP qui catalyse la ligature intramoléculaire.

6. Procédé selon l'une quelconque des revendications précédentes, dans lequel l'étape de digestion (v) est réalisée en utilisant une exoribonucléase 3'-5' ou une exoribonucléase 5'-3'.

7. Procédé selon l'une quelconque des revendications précédentes, dans lequel l'étape d'amplification circulaire roulante de transcription inverse (vi) est réalisée en utilisant une enzyme de transcription inverse choisie parmi des M MLV-RT modifiées (Transcriptase inverse du virus de la leucémie murine de Moloney) et des AMV-RT (Transcriptase inverse du virus de la myéloblastose aviaire).

8. Procédé selon l'une quelconque des revendications 2 à 6, dans lequel l'étape de génération du brin d'ADNc complémentaire (vii) est réalisée en utilisant une Polymérase Taq ayant une activité d'exonucléase 5'-3'.

9. Procédé selon l'une quelconque des revendications précédentes, dans lequel le lieur d'ARN aléatoire a une longueur comprise entre 50 et 500 nucléotides.

10. Procédé selon l'une quelconque des revendications précédentes, dans lequel le lieur d'ARN aléatoire a une énergie libre minimale comprise entre -3 et -150 kcal/mole.

11. Procédé selon l'une quelconque des revendications précédentes, dans lequel l'au moins une molécule d'ARN portant un groupe phosphate ou un groupe phosphate 2',3'-cyclique à l'extrémité 3' est présente de manière physiologique ou pathologique dans l'échantillon biologique, ou est générée en traitant l'échantillon biologique avec une endoribonucléase, une exoribonucléase, une ribozyme ou une toxine capable de cliver l'ARNm, l'ARNt, l'ARNsn, l'ARNsno, l'ARN Y, l'ARNlnc, l'ARNpi, l'ARNsi, l'ARN viral ou l'ARNr.

12. Kit comprenant un lieur d'ARN aléatoire et une première enzyme ligase, une exoribonucléase et éventuellement une deuxième enzyme ligase, dans lequel :
(i) le lieur d'ARN aléatoire porte un groupe -OH aux deux extrémités ;
(ii) l'enzyme ligase est appropriée pour ligaturer l'extrémité 3' d'une molécule d'ARN, portant un groupe phosphate ou un groupe phosphate 2',3'-cyclique à l'extrémité 3' et un groupe phosphate à l'extrémité 5', à l'extrémité 5' du lieur d'ARN aléatoire ;
(iii) l'exoribonucléase est appropriée pour digérer de manière enzymatique des molécules d'ARN linéaires ; et
(iv) la deuxième enzyme ligase est appropriée pour circulariser un produit de ligature obtenu par ligature du lieur d'ARN aléatoire à la molécule d'ARN.

13. Kit selon la revendication 12, dans lequel la première enzyme ligase est choisie parmi RtcB, Archéase, une ligase d'ARNt d'Arabidopsis Thaliana et une ligase d'ARNt d'eucaryote.

14. Kit selon la revendication 12 ou la revendication 13, dans lequel la deuxième enzyme ligase est choisie parmi T4 Rnl1, T4 Rnl2, T4 Rnl2tr, T4 Rnl2 K227Q, Mth Rnl et une ligase indépendante de l'ATP qui catalyse la ligature intramoléculaire.

15. Kit selon l'une quelconque des revendications 12 à 14, dans lequel le lieur d'ARN aléatoire a une longueur comprise entre 50 et 500 nucléotides.

16. Kit selon l'une quelconque des revendications 12 à 15, dans lequel le lieur d'ARN aléatoire a une énergie libre minimale comprise entre -3 et -150 kcal/mole.

17. Kit selon l'une quelconque des revendications 12 à 16, comprenant en outre (i) une enzyme phosphorylante, et/ou (ii) une endoribonucléase, un ribozyme ou une toxine capable de cliver l'ARNm, l'ARNt, l'ARNsn, l'ARNsno, l'ARN Y, l'ARNlnc, l'ARNpi, l'ARNsi, l'ARN viral ou l'ARNr.

18. Kit selon l'une quelconque des revendications 12 à 17, dans lequel le lieur d'ARN aléatoire a une séquence nucléotidique telle qu'exposée dans la SEQ ID NO: 3.
